# FASCICULE DE BREVET EUROPEEN

(11) **EP 0 572 635 B1**
(45) Date de publication et mention de la délivrance du brevet: **27.12.1996**
(21) Numéro de dépôt: 93901791.9
(22) Date de dépôt: 20.11.1992
(51) Int. Cl.: C07D 239/42, C07D 239/22, C07D 233/90, C07D 233/88, C07C 279/04, A61K 31/505, A61K 31/415, A61K 31/155

(54) **COMPOSES A STRUCTURE GUANIDIQUE ET COMPOSITIONS PHARMACEUTIQUES LES CONTENANT**
VERBINDUNGEN MIT GUANIDIN-STRUKTUR UND DIESE ENTHALTENDE PHARMAZEUTISCHE ZUSAMMENSETZUNGEN
COMPOUNDS HAVING A GUANIDINE STRUCTURE AND PHARMACEUTICAL COMPOSITIONS CONTAINING SAME

(30) Priorité: 22.11.1991 FR 9114414
(43) Date de publication de la demande: 08.12.1993
(73) Titulaire: Etablissement Public dit: CENTRE NATIONAL DE LA RECHERCHE SCIENTIFIQUE (CNRS), 75007 Paris (FR)
(72) Inventeur: THAL, Claude, F-92330 Sceaux (FR); QUIROSA-GUILLOU, Catherine Cité du Moulin de Grais, F-91370 Verrières-le-Bouisson (FR); POTIER, Pierre, F-75007 Paris (FR); RENKO, Dolor, F-91190 Gif-sur-Yvette (FR); ZANETTA, Jean-Pierre, F-67370 Griesheim (FR); PORTIER, Marie-Madeleine, F-91370 Verrières-le-Buisson (FR); SENSENBRENNER, Monique, F-67000 Strasbourg (FR); KOENIG, Janine, F-33800 Bordeaux (FR); KOENIG, Herbert, F-33800 Bordeaux (FR)
(74) Mandataire: Warcoin, Jacques
(86) Numéro de dépôt international: FR9201080
(87) Numéro de publication internationale: WO9310102

(56) Documents cités:
- EP-A- 526 877
- Chemical Abstracts, vol. 70, 1969, (Columbus, Ohio, US), E. BOSCHETTI et al.: "Synthesis and pharmacologic classification of new hypotensive agents", voir page 378, colonne 1, abrégé no. 68277w, & THERAPIE 1968, 23(5), 1221-32, voir abrégé (citée dans la demande)
- Chemical Abstracts, vol. 75, 1971, (Columbus, Ohio, US), C.M. GUPTA: "Novel class of hypoglecemic agents", voir page 450, colonne 1, abrégé no. 63742h, & INDIAN J. CHEM. 1971, 9(3), 201-06, voir abrégé
- Macromolecules, vol. 24, no. 23, 1991, (Easton, US), Y.-K. KIM et al.: "Synthesis and curing study of 5-5 ring fused polyimide based on imidazole", pages 6357-6360, voir page 6357; figure 3

## Description

La présente invention concerne de nouveaux composés à structure guanidinique favorisant la croissance, la réparation et la régénération de l'axone des neurones et susceptibles d'améliorer l'état des malades atteints de neuropathies et myopathies. Plus généralement, ces composés peuvent être utiles dans le domaine des maladies dégénératrices du système nerveux.

Elle a également pour objet les procédés de préparation permettant d'accéder auxdits composés et les compositions pharmaceutiques contenant ces composés.

A la connaissance du déposant, il n'existe pas de composés disponibles en thérapeutique humaine agissant sur la régénération du nerf périphérique et pouvant avoir un effet bénéfique sur les affections neuromusculaires.

Il a certes déjà été décrit dans "La Nouvelle Presse Médicale", Masson, 1982, 16 (11), 1193-1280 un composé, l'isaxonine ou 2-isopropylamine-pyrimidine, présentant des propriétés pharmacologiques très intéressantes : néanmoins, commercialisé sous le nom de Nerfactor, il a dû être retiré du marché pour cause d'hépatotoxicité.

Par ailleurs, dans la revue "Thérapie" (1968, XXIII, pp. 1221-1232) la vératrylguanidine, le 3,4-diéthoxy benzyle guanidine, le 3,4-n-propoxy benzyle guanidine et l'hémisulfate de ces composés ont éte décrits comme antihypertenseurs.

Toutefois, les propriétés de ces composés en neurothérapie n'ont, à la connaissance du déposant, jamais été décrites.

Enfin, la demande de brevet EP-A-526 877 non publiée à la date de priorité de la présente demande décrit le composé de formule :

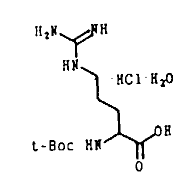

L'invention a donc pour objet de proposer de nouveaux composés, utiles dans le domaine de la régénération des nerfs, destinés par conséquent à avoir des effets bénéfiques dans les maladies y relatives telles que la myopathie.

Selon l'invention, les composés répondent à la formule: 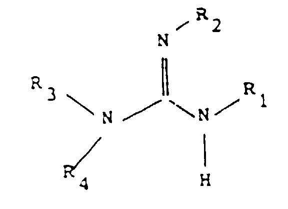 dans laquelle :
R₁ est un radical isopropyle, benzyle éventuellement substitué par un ou plusieurs radicaux (C₁-C₄) alcoxy ou un radical : 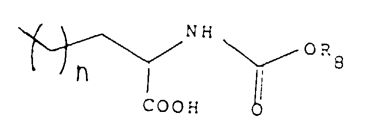
n = 0, 1, 2,
R₂, R₃ et R₄ ensemble avec les atomes d'azote auxquels ils sont attachés et l'atome de carbone auquel lesdits atomes d'azote sont rattachés forment un cycle pyrimidine de formule II ou pyrimidinium de formule IIa : 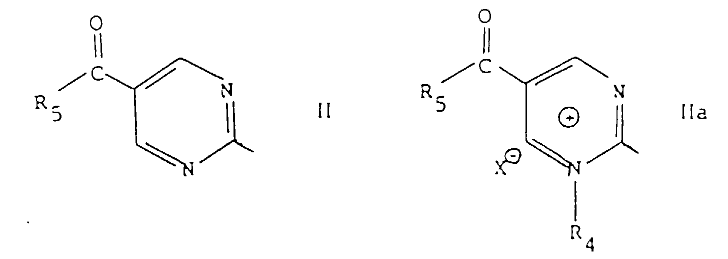
dans laquelle :
R₄ est un radical (C₁-C₄) alkyle, (C₇-C₉) aralkyle, phényle ou un atome d'hydrogène,
R₈ est un radical (C₁-C₄) alkyle, (C₇-C₉) aralkyle,
R₅ est un radical hydroxy, (C₁-C₄) alcoxy, amino,
X⁻ est un cation pharmacologiquement acceptable,
ou R₂, R₃, R₄ sont l'atome d'hydrogène,
ou R₂, R₃, R₄ ensemble forment avec les atomes d'azote auxquels ils sont rattachés et l'atome de carbone auxquels lesdits atomes d'azote sont rattachés un cycle imidazole de formule : 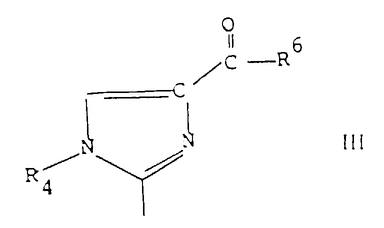 ou 1,6-dihydropyrimidine de formule : 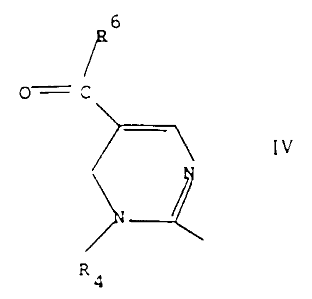
R₆ ayant l'une des significations de R₅,
ou R₂, R₃ ensemble forment avec les atomes d'azote auxquels ils sont rattachés et l'atome de carbone auxquels lesdits atomes d'azote sont rattachés un cycle de formule : 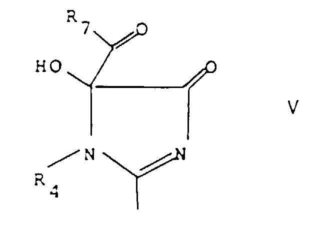
R₇ étant un radical (C₁-C₄) alkoxy ou 1-glycéryl avec dans ce dernier cas R₁ qui peut correspondre à l'atome d'hydrogène,
et les sels pharmacologiquement acceptables de ces composés à l'exception de la vératrylguanidine et de l'hémisulfate de celle-ci et des composés de formule I ou R₂, R₃, R₄ sont l'atome d'hydrogène et R₁ est un radical benzyle substitué en meta-para simultanément par deux radicaux éthoxy ou deux radicaux n-propoxy et les hémisulfates de ceux-ci, et à l'exception du composé ou R₂, R₃,R₄ sont l'atome d'hydrogène et R₁ est -(CH₂)₃-CH(COOH)-NH-COOtBu.

L'invention concerne également les sels pharmacologiquement acceptables de ces composés, notamment les lactate, fumarate, chlorhydrate maléate, malate, cétoglutarate, glutarate, phénoxyatcétate, sulfonate, picrate, tartrate, méthanesulfonate.

Selon une variante préférée de l'invention, R₁ est un radical isopropyle ou di- ou tri- méthoxybenzyle où le radical:

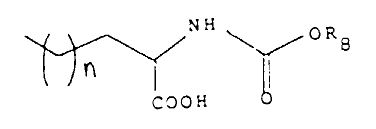

Selon une seconde variante préférée de l'invention, prise ou non en combinaison avec la précédente, R₄ est un radical méthyle, éthyle, n-propyle ou benzyle.

Selon une troisième variante préférée, l'invention concerne les lactate, fumarate, chlorhydrate, maléate, malate, cétoglutarate, glutarate, phénoxyacétate, sulfonate, picrate, tartrate, méthane sulfonate de vératrylguanidine.

L'invention concerne également les médicaments consistant en un des composés selon l'invention, tels qu'ils viennent d'être décrits ci-avant et les compositions pharmaceutiques contenant au moins un de ces médicaments et un support acceptable. Ces médicaments et compositions sont utiles pour le traitement médical pour l'homme ou les animaux de certains troubles liés au fonctionnement du système nerveux.

Ces médicaments et compositions peuvent ainsi être avantageusement destinés à participer à une thérapie efficace contre les maladies dégénératives du système nerveux périphérique et contre les affections neuromusculaires telles que la myopathie.

Les compositions pharmaceutiques sont notamment formulées pour être ingérées oralement ou pour être injectées. Néanmoins, d'autres modes d'administration peuvent également être envisagés dans le cadre de la présente invention.

La posologie dépendra pour partie de la maladie à traiter ainsi que de sa gravité et également dsu type de l'individu (poids, âge).

Pour les composés déjà connus comme le vératrylguanidine ou l'hémisulfate de celui-ci ou les composés de formule I ou R₂, R₃, R₄ sont l'atome d'hydrogène et R₁ est un radical benzyle substitué en meta-para simultanément par deux radicaux éthoxy ou deux radicaux n-propoxy et les hémisulfates de ceux-ci, l'invention a pour objet l'utilisation de ces composés pour la fabrication d'un médicament utile pour le traitement des neuropathies ou des myopathies.

L'invention concerne également des procédés de préparation des composés selon l'invention.

Un procédé de préparation des composés de formule (I) dans laquelle R₂, R₃ et R₄ ensemble avec les atomes d'azote auxquels ils sont attachés et l'atome de carbone auquels lesdits atomes d'azote sont rattachés forment un cycle pyrimidine de formule II, consiste à mettre en contact un sel acide de guanidine (par exemple l'hydrogénosulfate) de formule : 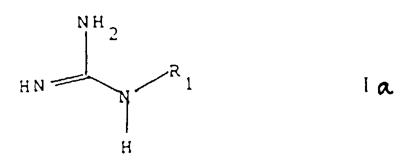 avec un (C₁-C₄) alkoxycarbonylmalonaldéhyde de formule : 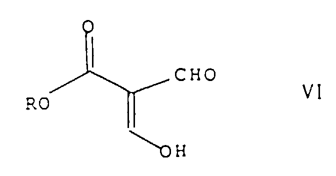 en présence d'un accepteur d'acide (comme une amine secondaire telle que la pyrrolidine) et éventuellement d'un solvant polaire protique tel que le méthanol.

Les composés obtenus sous forme non salifiée sont ensuite transformés en acide par hydrolyse de manière connue, et éventuellement transformés en carboxamide de manière connue également (par exemple par réaction avec l'aminochlorométhylaluminium.

Par ailleurs, l'alkylation de ces pyrimidines de formule Il sous quelque forme que ce soit (acide, ester, amide) peut être effectuée par action d'un sulfate de dialkyle en solvant polaire aprotique pour conduire au sulfate acide purifié sous forme d'hydroxyde de 1-alkyl pyrimidinium correspondant qui peut être ensuite transformé en un autre sel (par exemple le sel de l'acide méthane sulfonique).

L'hydroxyde formé peut également être transformé en 1,6-dihydro 1-alkyl pyridimine de formule IV par réaction avec un agent réducteur, notamment avec le tétraborohydrure de sodium en présence éventuellement d'un solvant polaire protique (notamment l'éthanol).

Un procédé de préparation des composés de formule (I) dans laquelle R₂, R₃ et R₄ ensemble forment avec les atomes d'azote auxquels ils sont rattachés et l'atome de carbone auxquels lesdits atomes d'azote sont rattachés un cycle imidazole de formule III consiste essentiellement à décarboxyler partiellement les 4,5-dicarboxy imidazoles correspondants par chauffage éventuellement en présence d'un solvant polaire comme le N,N-diméthylacétamide.

Les 4,5-dicarboxy imidazoles sont obtenus par saponification des 4,5-dicyano correspondants de manière connue. Ces derniers sont soit des produits commerciaux soit obtenus de manière connue.

Un procédé de préparation par exemple consiste à alkyler en position 1- le 2-bromo 4,5-dicyanoimidazole par réaction avec un dialkyl sulfonate. Ce dernier composé est substitué en position 2- par une amine H₂N-R₁ puis traité par une base forte (par exemple NaOH) et acidifié (par exemple HCl concentré).

Le 2-bromo 4,5-dicyanoimidazole de départ est obtenu de manière connue par bromation du 4,5-dicyanoimidazole.

Un autre procédé de préparation d'imidazoles selon l'invention dans lesquels R₄ est l'atome d'hydrogène (donc avec présence d'une tautomérie) consiste à effectuer la suite de réactions selon le schéma suivant :

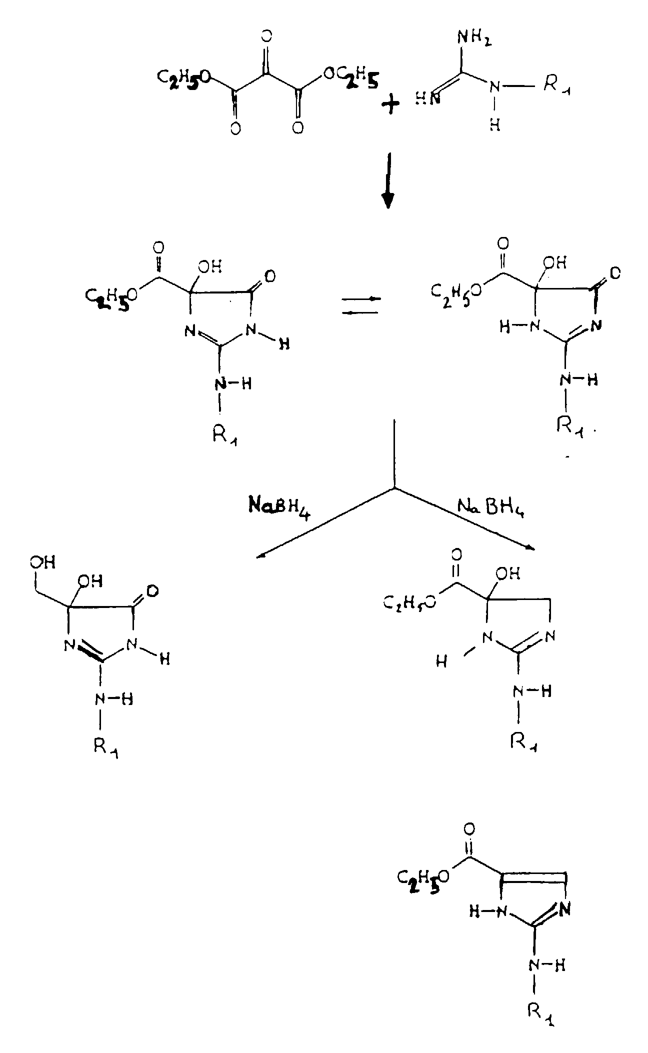

Un procédé de préparation des guanidines de formule (Ia) ou de leurs sels pharmacologiquement acceptables consiste à faire réagir une S-alkylthiourée avec une alkylamine R₁NH₂ de manière connue.

Un procédé de fabrication des composés de formule (I) dans laquelle R₂, R₃ ensemble forment avec les atomes d'azote auxquels ils sont rattachés et l'atome de carbone auxquels lesdits atomes d'azote sont rattachés un cycle de formule V, où R₇ est un radical (C₁-C₄) alkoxy, consiste à faire réagir la guanidine de formule la correspondante avec un dialkylcétomalonate éventuellement en milieu protique polaire selon le schéma réactionnel ci-avant.

Dans le cas où R₇ est le radical 1-glycéryl, un procédé de préparation consiste à condenser une guanidine de formule (Ia) sur la forme oxydée de l'acide ascorbique, selon le schéma réactionnel suivant :

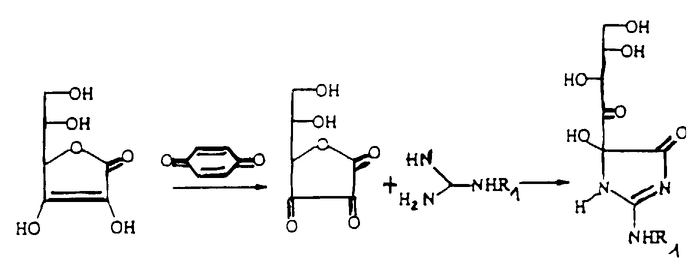

Les exemples ci-après illustrent l'invention :

### Exemple 1 :

### 1-méthyl-2-isopropylamino-5-méthoxycarbonyl-1,6-dihydropyrimidine

A 10,5 g (32 mmol) d'hydroxyde de 1-méthyl-2-isopropylamino-5-méthoxycarbonyl-pyrimidinium dans 180 ml d'éthanol anhydre, on additionne par portion sous argon à -40°C du borohydrure de sodium (2,8 g ; 73 mmol). Le mélange est agité sous argon pendant une heure à température ambiante. Après évaporation de l'éthanol, le milieu réactionnel est extrait avec du dichlorométhane et de l'eau. Les phases organiques sont lavées avec une solution saturée en chlorure de sodium puis séchées sur sulfate de sodium. La purification sur gel de silice (200 mb ; éluant : MeOH 10 % / CH₂Cl₂) de l'huile obtenue par évaporation des phases organiques conduit à 7 g (Rdt : 100 %) d'ester dihydro correspondant.
Point de fusion : 83°C.

L'hydroxyde de 1-méthyl-2-isopropylamino-5-méthoxycarbonyle pyrimidinium est obtenu de la façon suivante :

Un mélange de 27,45 g (0,140 mol) de 2-isopropylamino-5-méthoxycarbonyle pyrimidine et de 56 ml (2 éq ; 0,59 mol) de sulfate diméthylique dans 440 ml de tétrahydrofurane anhydre est porté au reflux pendant 24 heures. Le milieu réactionnel est extrait avec de l'acétate d'éthyle et de l'eau. La purification sur gel de silice (éluant : CH₂Cl₂95 / MeOH 5 % / saturation avec NH₃ gaz) de l'huile résultant de l'évaporation des phases aqueuses conduit à 37,2 g d'hydroxyde de 1-méthyl-2-isopropylamino-5-méthoxycarbonyle pyrimidinium (rdt : 82 %).

### Exemple 2

### 2-isopropylamino-5-méthoxycarbonyl-pyrimidine

Un mélange de 11.62 g (77,5 mmol) d'hydrogénosulfate de l'isopropylguanidine, de 32 ml (5éq ; 0,37 mol) de pyrrolidine fraîchement distillée dans 230 ml de méthanol anhydre est porté au reflux pendant vingt minutes. Une solution méthanolique (60 ml) de méthoxycarbonylmalonaldéhyde (1 éq ; 77,5 mmol ; 10 g) est additionnée à la solution précédente à 0°C ainsi que 140 g de tamis moléculaire (4 A°). Le milieu réactionnel est agité sous argon au reflux durant 24 heures. Les tamis moléculaires sont éliminés par filtration puis rincés avec du méthanol. L'évaporation du filtrat conduit à un résidu qui est repris avec de l'acétate d'éthyle puis acidifié avec une solution d'acide chlorhydrique IN. Les phases organiques sont lavées avec une solution saturée en chlorure de sodium, séchées sur sulfate de sodium, évaporées et purifiées sur gel de silice sous 200 mb (éluant : acétate d'éthyle 50/heptane 50).
Poids : 11,27 g (rdt : 75 %).
Point de fusion 102°C.

### Exemple 3

### Sulfate de 1-méthyl-2-isopropylamino-5-méthoxycarbonyl-pyrimidinium

1,09 mlg (16,78 mol ; 1,61 g) d'acide méthane sulfonique est additionné à une solution d'hydroxyde de 1-méthyl-2-isopropylamino-5-méthoxycarbonyl-pyrimidinium (3,48 g; 15,35 mol) dissous dans 100 ml de tétrahydrofurane anhydre. Le mélange est agité sous argon à température ambiante durant une heure. Le précipité qui se forme est filtré puis rincé avec de l'éther.
Poids : 3,74 g (rdt : 80 %).
Point de fusion : 209°C.

### Exemple 4

### 2-isopropylamino-5-carbamoyl-pyrimidine

30,6 ml (61,2 mmol) d'une solution 2N de triméthylaluminium dans le toluène sont lentement introduits dans une suspension de chlorure d'ammonium (61,2 mmol ; 3,26 g) dans 71 ml de benzène anhydre à 0°C. L'addition terminée, le mélange est agité sous argon durant une à deux heures jusqu'à épuisement du dégagement de méthane.

A 92 ml de la solution précédente (6 éq de réactif), une solution de 2-isopropylamino-5-méthoxycarbonyl-pyrimidine (0,102 mol ; 2 g) dans 40 ml de benzène anhydre est additionnée avec un jonc. Après 48 heures d'agitation à température ambiante sous argon, le mélange est neutralisé avec de l'acide chlorhydrique à 5 %, puis extrait avec du dichlorométhane. Les phases organiques sont éliminées, les phases aqueuses filtrées et évaporées. L'amide sous forme de poudre est obtenu avec un rendement de 80 % après purification sur gel de silice (éluant : CH₂Cl₂ 80 % / MeOH 20 % / 0,7 % de NH₃ en solution aqueuse à 33 %).

### Exemple 5

### 2-isopropylamino-5-carboxy-pyrimidine

6 ml d'une solution de soude 1N sont ajoutés à une solution de 2-isopropylamino-5-méthoxycarbonyl-pyrimidine (1 g ; 5,12 mmol) dans 33 ml de méthanol. Le mélange est porté au reflux (66°C) pendant deux heures. Un minimum d'eau est additionné afin de dissoudre le précipité qui se forme ; le méthanol est évaporé sous pression réduite. La solution restante est acidifiée à 0°C avec de l'acide chlorhydrique 2N. Le précipité obtenu est filtré, lavé à l'eau, puis séché pour conduire à 850 mg (92 %) d'acide.

### Exemple 6

### 1-méthyl-2-isopropylamino-5-carbamoyl-1,6-dihydropyrimidine

49,7 ml (99,5 mmol) d'une solution 2N de triméthylaluminium dans le toluène sont lentement introduits dans une suspension de chlorure d'ammonium (99,5 mmol ; 5,32 g) dans 105 ml de toluène anhydre à 0°C. L'addition terminée, le mélange est agité sous argon durant une à deux heures jusqu'à épuisement du dégagement de méthane.

Une solution de 1-méthyl-2-isopropylamino-5-méthoxycarbonyle-1,6-dihydropyrimidine (3,4 g ; 0,16 mol) dans 175 ml de toluène anhydre est additionnée avec un jonc à 144 ml de la solution 0,67 M d'aminochlorométhyl-aluminium (6 éq de réactif). Après 60 heures d'agitation à température ambiante sous argon, le mélange est lentement neutralisé à 0°C avec de l'acide chlorhydrique 1N (350 ml), puis extrait avec de l'acétate d'éthyle. Les phases aqueuses sont évaporées, reprises avec de l'éthanol saturé en ammoniac et de la silice (qui retient les sels d'aluminium) ; ce mélange est filtré puis évaporé. Après plusieurs flash-chromatographies sur gel de silice (200 mb ; MeOH 5- 30 % / CH₂Cl₂/NH₃ saturation) du filtrat, 2,28 g d'amide sont obtenus avec un rendement de 72 %.
Point de fusion : 179-180°C.

### Exemple 7

### α-N-Boc-δ-N-(5-méthoxycarbonyl-2-pyrimidinyl)-L-ornithine

Le mélange de 4 g (14,5 mmol) de L-arginine alpha-N-Boc (commercial) de 2,84 g (1,5 eq ; 21,8 mol) de méthoxycarbonylmalonaldéhyde, de 3,65 ml de pyrrolidine fraîchement distillée, de 10 g de tamis moléculaire 4 A° activé, dans 30 ml de méthanol anhydre est porté à 50°C pendant 24 heures sous argon. Après élimination, des tamis moléculaires par filtration et évaporation de méthanol, le milieu réactionnel est extrait avec de l'eau et du dichlorométhane. Les phases aqueuses sont évaporées puis purifiées sur gel de silice (200 mbar ; éluant : CH₂Cl₂ 80 % / MeOH 20 % / NH₃ en solution aqueuse à 33 % 0,7 ml pour 100 ml). On obtient 4,21 g (rdt : 80 %).

### Exemple 8

### 2-vératrylamino-5-carboxy pyrimidine

0,4 ml d'une solution de soude 1N est ajouté à une solution méthanolique (1 ml) de 2-vératrylamino-5-méthoxycarbonyl-pyrimidine (100 ml ; 3,3 mmol). Le mélange est porté au reflux pendant deux heures. Le méthanol est évaporé sous pression réduite. La solution restante est acidifiée avec de l'acide chlorhydrique 2N ; le précipité qui se forme est filtré, lavé avec de l'eau puis séché (rdt : 90 %).

### Exemple 9

### 2-vératrylamino-5-méthoxycarbonyl-pyrimidine

Un mélange de 21,92 g (0,1 mmol) de vératrylguanidine obtenu selon "Thérapie" (op.cit.), de 41,7 ml (0,5 mol) de pyrrolidine fraîchement distillée, de 15 g de méthoxycarbonylmalonaldéhyde, de 180 g de tamis moléculaire (4 A°) dans 300 ml de méthanol anhydre est porté au reflux sous argon durant vingt quatre heures. Les tamis moléculaires sont éliminés par filtration, puis rincés avec du méthanol et du chloroforme. L'évaporation du filtrat conduit à un résidu qui est repris avec de l'acétate d'éthyle puis acidifié avec une solution d'acide chlorhydrique 1N. Les phases organiques sont lavées avec une solution saturée en chlorure de sodium, séchées sur sulfate de sodium, évaporées et purifiées sur gel de silice (éluant : acétate d'éthyle 50/heptane 50) (rdt : 40 %).

### Exemple 10

### 1-méthyl-2-vératrylamino-5-méthoxycarbonyl-1,6-dihydropyrimidine

0,170 g (3 eq) de borohydrure de sodium est additionné à 0,400 g d'hydroxyde de 1-méthyl-2-vératrylamino-5-méthoxycarbonyl-pyrimidinium dissous dans 50 ml d'éthanol. Le mélange est agité sous argon à température ambiante durant trois heures. Après évaporation de l'éthanol, le milieu est extrait avec du dichlorométhane et de l'eau. Les phases organiques sont lavées avec une solution saturée en chlorure de sodium puis séchées sur sulfate de sodium. L'huile résultante de l'évaporation des phases organiques purifiée sur gel de silicie sous pression moyenne (éluant : MeOH 15 % / CH₂Cl₂) conduit à 467 mg (rdt : 98 %) de dihydro-1,6 pyrimidine correspondante.

### Exemple 11

### Hydroxyde de 1-méthyl-2-vératrylamino-5-méthoxycarbonyl-pyrimidinium

Un mélange de 2,62 g (8,6 mmol) de 2-vératryl amino-5-méthoxycarbonyl-pyrimidine et de 3.28 ml (4 éq) de sulfate diméthylique dans 60 ml de THF anhydre est porté au reflux pendant quarante huit heures. Le milieu est extrait avec de l'acétate d'éthyle et de l'eau. La purification sur gel de silice (éluant : MeOH 3 % / CH₂Cl₂ / NH₃ en solution aqueuse à 33 % 5,5 ml/100) de l'huile résultant de l'évaporation des phases aqueuses conduit à 2,2 g de sel (rdt : 76 %).

### Exemple 12

### Sulfate de 1-méthyl-2-vératrylamino-5-méthoxycarbonyl-pyrimidinium

A 400 mg d'hydroxyde de 1-méthyl-2-vératrylamino-5-methoxycarbonyl-pyrimidinium dissous dans 15 ml de tétrahydrofurane anhydre. on additionne 85 µl (1.1 eq ; 1,31 mmol) d'acide méthane sulfonique. Le mélange est agité sous argon, à température ambiante pendant une heure. Le précipité est filtré, rincé avec du tétrahydrofurane et de l'éther, puis séché. (poids : 490 mg - Rdt : 96 %).

### Exemple 13

### Méthanesulfonate de vératrylguanidine

A une suspension de vératrylguanidine (25 g ; 0,119 mol) dans 240 ml de méthanol on introduit (7,8 ml ; 0,120 mol) d'acide méthane sulfonique. Le mélange est agité à température ambiante pendant une heure. Le méthanol est évaporé sous vide, l'huile obtenue est précipitée par agitation pendant 15 min dans le tétrahydrofurane. Le solide blanc est filtré, rincé avec du tétrahydrofurane et de l'éther puis séché sous vide. 30 g de méthanesulfonate de vératrylguanidine sont obtenus avec un rendement de 82 %.

### Exemple 14

### 1-méthyl-2-isopropylamino-4-carboxy-imidazole

Un mélange de 1-méthyl-2-isopropylamino-4,5-dicarboxy-imidazole (910 mg ; 4 mmol) et de 20 ml de N,N diméthylacétamide est chauffé à 180°C sous argon pendant trois heures. Le solvant est évaporé sous pression réduite ; le résidu repris avec le minimum d'éthanol est précipité avec du tétrahydrofurane. Les eaux mères sont concentrées jusqu'à précipitation. Le 1-méthyl-2-isopropylamino-4-carboxyimidazole est obtenu avec un rendement de 88 %.
(m = 650 mg).
Point de fusion : 247°C.

### Exemple 15

### 1-méthyl-2-isopropylamino-4-méthoxycarbonyl-imidazole

400 mg de 1-méthyl-2-isopropylamino-4-carboxy-imidazole sont solubilisés dans 25 ml de méthanol anhydre. La solution est refroidie à 0°C. saturée en acide chlorhydrique, puis portée au reflux durant douze heures. Après refroidissement l'excès d'acide est chassé par un courant d'argon. Le milieu est neutralisé par addition de carbonate de sodium et évaporé. Le résidu repris avec de l'eau est extrait avec du dichlorométhane. Les phases organiques séchées sur sulfate de sodium, évaporées conduisent à un résidu qui est lavé avec de l'éther puis séché sous vide.
m = 384 mg ; Rdt : 89 %.
Point de fusion : 198°C.

### Exemple 16

### 2-isopropylamino-4-oxo-5-éthoxycarbonyl-5-hydroxy-Δ₂-imidazoline

5,72 g (0,0191 mol) d'hydrogénosulfate de l'isopropylguanidine sont dessalifiés par une solution méthanolique (20 ml) de soude (0,038 mol ; 1,53 g) à température ambiante sous argon pendant une heure. Le précipité résultant est filtré ; le filtrat évaporé. L'isopropylguanidine est reprise avec de l'éthanol et de nouveau filtrée. Le filtrat éthanolique est concentré.

3,85 g (0,0382 mol) d'isopropylguanidine et de 10 g de diéthylcétomalonate dans 150 ml d'éthanol sont chauffés pendant 10 heures à 50°C. L'évaporation de l'éthanol conduit à un résidu qui est purifié par flash-chromatographie sur gel de silice 60 (éluant : MeOH 15 / CH₂Cl₂) (m = 7 g ; rdt : 80 %). Le composé existe sous deux formes en équilibre A et B.

### Exemple 17

### 2-amino-4-(2'3'4'-trihydroxybutanoyl)-4-hydroxy-5-oxo-imidazoline

Un mélange de L-acide ascorbique (0,314 mol ; 55,4 g) et de p-benzoquinone dans 470 ml d'éthanol est agité dans le noir sous argon pendant 90 min ; puis une solution éthanolique (100 ml) de guanidine (0.157 mol) (préalablement dessalifiée avec de la soude dans l'éthanol) est alors ajoutée. Le milieu est agité à température ambiante sous argon dans le noir durant 6 h. Le précipité qui se forme est filtré, lavé avec de l'éthanol et séché sous vide. (m = 30 g ; rdt = 100 %).

On a également obtenu selon une technique connue l'isopropylguanidine (exemple 18), l'isaxonine (exemple 19), le chlorure d'isopropyl guanidine (exemple 20).

### Essais biologiques

### A - Effet sur la poussée neuritique des ganglions spinaux

### Mode expérimental :

On met en culture des ganglions prélevés sur des rats nouveaux nés dans des plaques de 96 puits à fond plat dans un milieu DMEM (Gibco) auquel sont ajoutés 5 % de sérum fétal de veau (FCS) et 10 mM d'Ara-C (Cytosine-β-D- arabinofuranoside).

Après un jour, on ajoute les différents composés à tester et des solutions témoins.

Deux jours après, on arrête la culture et on photographie des explants à faible grossissement au microscope à contraste de phase, ou en microscopie classique après coloration par le bleu de toluidine après fixation par des mélanges d'aldéhydes. Sont pris en compte dans les évaluations le diamètre moyen d'extension des faisceaux neuritiques, leurs ramifications, leur association avec les cellules gliales et les morts cellulaires.

### Composés testés :

Les composés 1 à 7, 13, 17 et 19 ont été testés à trois concentrations différentes : 10⁻⁵, 10⁻⁶ et 10⁻⁷ M en présence ou en absence de 50 Ul/ml de NGF (sigle anglo-saxon pour désigner le facteur de croissance nerveux). Les expériences en présence de NGF avalent pour but, d'une part la comparaison avec les produits testés, d'autre part l'observation d'une éventuelle modification de l'effet NGF (mort cellulaire, induction de ramifications neuritiques).

Sans additif autre que le PBS, qui est utilisé pour solubiliser les divers composés, on observe la présence de rares prolongements courts et d'un nombre assez important de cellules mortes. En présence du témoin NGF, on note de nombreux prolongements fins et allongés peu ramifiés.

L'effet des différents produits est évalué par comparaison avec le témoin de référence : produit seul par rapport au PBS, d'une part, produit-NGF par rapport au NGF, d'autre part. Par ailleurs, l'effet positif d'un produit utilisé seul est comparé à celui du NGF.

Le composé de l'exemple 3 en présence de NGF provoque une modification importante des neurites ; ceux-ci sont plus longs et surtout présentent une arborisation importante avec de nombreuses cellules accolées aux prolongements. Le composé de l'exemple 13 en présence de NGF provoque un allongement des neurites (le double environ des cultures en présence de NGF seul). Il y a également un effet favorisant la réticulation des neurites (neurites moins nombreux de diamètre plus grand).

Les composés 3 et 13 employés seuls accentuent la poussée neuritique par rapport au témoin PBS. L'aspect morphologique des prolongements est identique à ceux du NGF et l'efficacité semble similaire à celle de ce facteur de croissance.

Le composé de l'exemple 5 utilisé seul à faible concentration ou en présence de NGF (10⁻⁷ M) présente un effet positif avec augmentation du nombre de prolongements.

Les composés des exemples 7 et 20 présentent un effet positif mis en évidence seul ou en synergie avec le NGF et caractérisé par une augmentation de la longueur des neurites.

Avec les exemples 2, 6 et 19, on remarque la présence d'un plus grand nombre de prolongements sans augmentation de leur longueur.

### B - Effet sur les neurones et les cellules gliales de poulet et de rat en culture primaire

Les composés des exemples 13 et 19 ont été testés sur la survie et la croissance des neurites de neurones des systèmes nerveux central et périphérique en culture primaire, ainsi que sur la prolifération et la modification morphologique des astrocytes.

### Mode expérimental :

**1. Neurones de cerveau d'embryon de rat de 14 jours**
   Les neurones sont dissociés à partir d'hémisphères cérébraux et sont cultivés sur polylysine dans des boîtes de Pétri en présence de milieu nutritif sans sérum chimiquement défini.
**2. Neurones de ganglions spinaux et ciliaires d'embryon de poulet de 8 jours**
   Les neurones dissociés sont cultivés sur polyornithine dans un milieu nutritif contenant 20 % de sérum de veau foetal.
**3. Astrocytes de cerveau de rat nouveau-né**
   Les cellules dissociées sont cultivées directement sur la surface du plastique de la boîte de Pétri en milieu défini.

Les composés 13 et 19 ont été ajoutés aux concentrations de 10⁻⁴ à 10⁻⁸ M dès la mise en culture et à chaque changement de milieu.

Sur les neurones isolés en culture pure, le composé 19 stimule la croissance neuritique des neurones du système nerveux central et le composé 13 favorise la survie et la croissance neuritique des neurones des ganglions spinaux (système nerveux périphérique). De plus, le composé 13 n'a pas d'effet toxique sur les cellules de soutien (astrocytes). Ces composés, susceptibles de réguler la survie neuronale et la croissance neuritique, constituent une voie thérapeutique dans les neuropathies et myophathies.

### C - Effet neurotrophique dans l'axe neuromusculaire

Les composés 1, 3, 13 ont été testés in vivo sur la régénération des axones et leur remyélinisation chez la souris Trembler, modèle animal de la neuropathie de Charcot-Marie-Tooth chez l'homme.

Les composés 1 en injection sous-cutanée (100 mg/kg) et 13 en injection intrapéritonéale (50 mg/kg) favorisent le "sprouting" axonal intranerveux (+ 25 % et + 20 %) et ils accélèrent la démyélinisation qui caractérise la mutation Trembler (- 25 %).

Le composé 3 favorise également la démyélinisation. Après 40 jours d'injections quotidiennes, 22 % seulement des fibres nerveuses sont myélinisées, au lieu de 32 % chez les témoins.

Les composés 1, 3, 7 et 13 ont été essayés in vitro à 10⁻³ M, 10⁻⁵ M et 10⁻⁷ M dans les cultures suivantes :
1) cellules de la moelle épinière d'embryons de rats âgés de 14 jours ;
2) myoblastes de souris normales et Mdx, modèle animal proche de la myopathie de Duchenne chez l'homme
3) co-cultures de myoblastes (embryons de rats de 19 jours) et de neurones (embryons de rats de 14 jours) et
4) cellules 3T3, lignée fibroblastique.

Les composés 1, 3, 13 favorisent la formation d'amas cellulaires. l'émergence et la pousse des neurites. Le composé 13 est le plus actif des trois.

Dans les co-cultures neurones-muscles, les composés 3 et 13 favorisent la pousse neuritique et leur ramification.

Dans les cocultures, les jonctions neuromusculaires (synapses) sont détectées par la co-localisation de l'acétyl-cholinestérase et des récepteurs de l'acétylcholine.

Le composé 7 augmente le nombre de synapses de 60 % à 10⁻³ M.

L'acroissement du nombre de synapses formées sous l'influence du composé 7 est dose-dépendante.

## Revendications

1. Composés de formule : 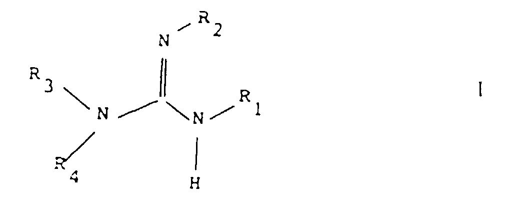 dans laquelle :
R₁ est un radical isopropyle, benzyle éventuellement substitué par un ou plusieurs radicaux (C₁-C₄) alcoxy ou un radical : 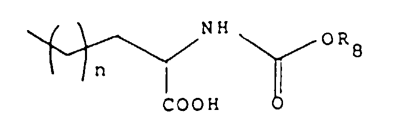
R₂, R₃ et R₄ ensemble avec les atomes d'azote auxquels ils sont attachés et l'atome de carbone auquel lesdits atomes d'azote sont rattachés forment un cycle pyrimidine de formule II ou pyrimidinium de formule IIa : 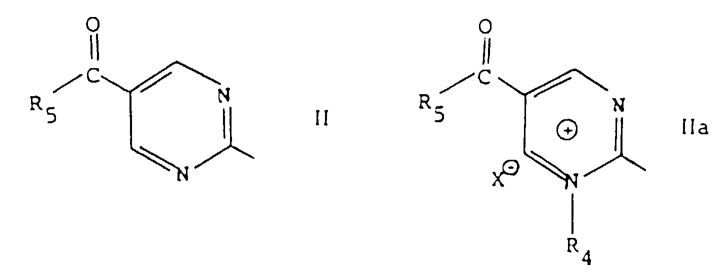
dans laquelle :
n = 0, 1, 2,
R₄ est un radical (C₁-C₄) alkyle, (C₇-C₉) aralkyle, phényle ou un atome d'hydrogène,
R₈ est un radical (C₁-C₄) alkyle, (C₇-C₉) aralkyle,
R₅ est un radical hydroxy, (C₁-C₄) alcoxy, amino,
X⁻ est un cation pharmacologiquement acceptable,
ou R₂, R₃, R₄ sont l'atome d'hydrogène,
ou R₂, R₃, R₄ ensemble forment avec les atomes d'azote auxquels ils sont rattachés et l'atome de carbone auxquels lesdits atomes d'azote sont rattachés un cycle imidazole de formule : 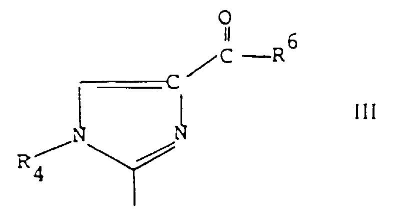
ou 1,6-dihydropyrimidine de formule : 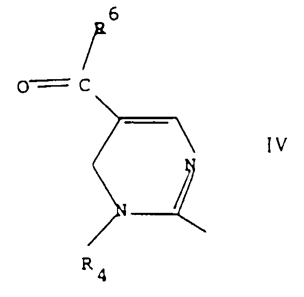
R₆ ayant l'une des significations de R₅,
ou R₂, R₃ ensemble forment avec les atomes d'azote auxquels ils sont rattachés et l'atome de carbone auxquels lesdits atomes d'azote sont rattachés un cycle de formule : 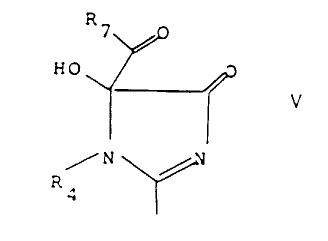
R₇ étant un radical (C₁-C₄) alkoxy ou 1-glycéryl avec dans ce dernier cas R₁ qui peut correspondre à l'atome d'hydrogène,
et les sels pharmaceutiquement acceptables de ces composés,
à l'exception de la vératrylguanidine et de l'hémisulfate de celle-ci et des composés de formule I où R₂, R₃, R₄ sont l'atome d'hydrogène et R₁ est un radical benzyle substitué en meta-para simultanément par deux radicaux éthoxy ou deux radicaux n-propoxy et des hémisulfates de ceux-ci,
et à l'exception du composé où R₂, R₃, R₄ sont l'atome d'hydrogène et R₁ est -(CH₂)₃-CH(COOH)-NH-COOtBu.

2. Composés selon la revendication 1, caractérisés en ce que R₁ est un radical isopropyle ou di- ou tri- méthoxybenzyle ou le radical :

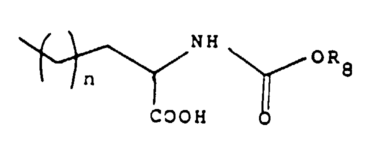

3. Composés selon la revendication 1, caractérisés en ce que R₄ est un radical méthyle, éthyle, n-propyle ou benzyle.

4. Composés selon l'une des revendications 1 à 3, caractérisés en ce qu'ils répondent à la formule 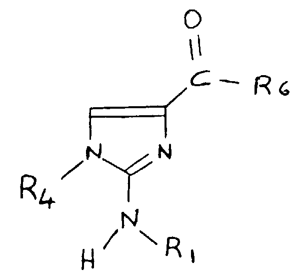 ainsi que les sels pharmaceutiquement acceptables de ces composés.

5. Composé selon la revendication 4, caractérisé en ce qu'il s'agit du 1-méthyl-2-isopropylamino-4-carboxy-imidazole.

6. Composés selon la revendication 1, caractérisés en ce qu'ils sont choisis parmi les lactate, fumarate, chlorhydrate, maléate, malate, cétoglutarate, glutarate, phénoxyacétate, sulfonate, picrate, tartrate, méthane sulfonate de vératrylguanidine.

7. Composés de formule I selon l'une des revendications 1 à 6, et le composé de formule I où R₂, R₃, R₄ sont l'atome d'hydrogène et R₁ est -(CH₂)₃-CH(COOH)-NH-COOtBu pour leur application en tant que substance thérapeutiquement active.

8. Composés de formule I selon la revendication 7, caractérisés en ce qu'ils sont appliqués pour le traitement des neuropathies ou des myopathies.

9. Composition pharmaceutique contenant au moins un composé selon la revendication 7 ou 8 et un support inerte pharmacologiquement acceptable.

10. Utilisation de la vératrylguanidine ou de l'hémisulfate de celle-ci ou des composés de formule I où R₂, R₃, R₄ sont l'atome d'hydrogène et R₁ est un radical benzyle substitué en meta-para simultanément par deux radicaux éthoxy ou deux radicaux n-propoxy et les hémisulfates de ceux-ci pour la fabrication d'un médicament utile dans le traitement des neuropathies ou des myopathies.

11. Procédé de préparation des composés de formule I selon l'une des revendications 1 à 3, dans laquelle R₂, R₃, R₄ ensemble avec les atomes d'azote auxquels ils sont attachés et l'atome de carbone auquel lesdits atomes d'azote sont rattachés forment un cycle pyrimidine de formule Il, consistant à mettre en contact un sel acide de guanidine de formule 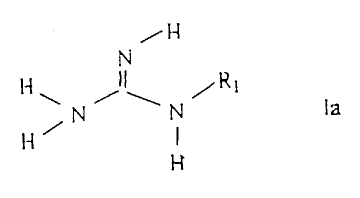 avec un (C₁-C₄) alkoxycarbonylmalonaldéhyde de formule : 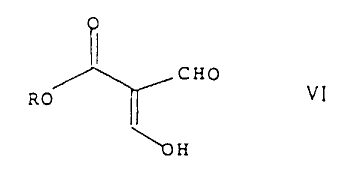 R est un (C₁-C₄) alkyle)
en présence d'un accepteur d'acide et éventuellement d'un solvant polaire protique, les composés obtenus pouvant être éventuellement hydrolysés et éventuellement transformés en carboxamide et/ou éventuellement en hydroxyde de 1-alkylpyrimidinium de formule IIa par action d'un sulfate de dialkyle puis ensuite transformés en un autre sel.

12. Procédé de préparation des composés de formule I, selon l'une des revendications 1 à 3, dans laquelle R₂, R₃, R₄ ensemble forment avec les atomes d'azote auxquels ils sont rattachés et l'atome de carbone auquel lesdits atomes d'azote sont rattachés, un cycle 1,6-dihydropyrimidine de formule IV, consistant dans le cas où R₄ est un radical alkyle à mettre en contact l'hydroxyde de pyrimidinium de formule : 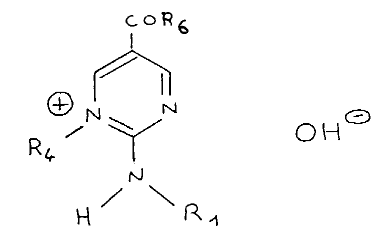 avec un agent réducteur comme le tétraborohydrure de sodium.

13. Procédé de préparation des composés de formule I selon la revendication 1, dans laquelle R₂, R₃ et R₄ ensemble forment avec les atomes d'azote auxquels ils sont rattachés et l'atome de carbone auquel lesdits atomes d'azote sont rattachés un cycle imidazole de formule III, caractérisé en ce qu'il consiste essentiellement à décarboxyler partiellement les 4,5-dicarboxy imidazoles correspondant aux composés de formule III par chauffage, éventuellement en présence d'un solvant polaire.

14. Procédé de préparation des composés de formule I selon la revendication 1, dans laquelle R₂, R₃ et R₄ ensemble forment avec les atomes d'azote auxquels ils sont rattachés et l'atome de carbone auquel lesdits atomes d'azote sont rattachés, un cycle imidazole de formule III où R₄ est un atome d'hydrogène, caractérisé en ce qu'il consiste essentiellement à déshydrater un composé de formule :

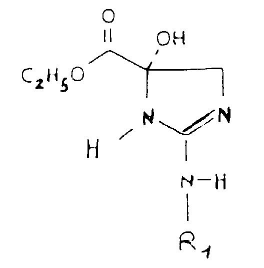

## Patentansprüche

1. Verbindungen der Formel 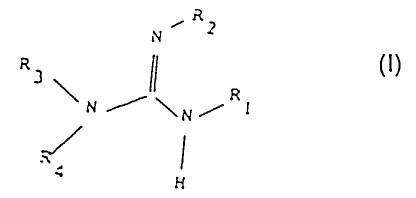 in der bedeuten:
R₁ einen Isopropylrest, einen Benzylrest, der gegebenenfalls substituiert ist durch einen oder mehrere (C₁-C₄)-Alkoxyreste, oder einen Rest 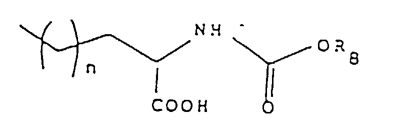 worin n für die Zahl 0, 1 oder 2 und R₈ für einen (C₁-C₄)-Alkyl- oder (C₇-C₉)-Aralkylrest stehen, und
R₂, R₃ und R₄ gemeinsam zusammen mit den Stickstoffatomen, an die sie gebunden sind, und dem Kohlenstoffatom, an das die genannten Stickstoffatome gebunden sind, einen Pyrimidin-Ring der Formel (II) oder einen Pyrimidiniumring der Formel (lla) bilden: 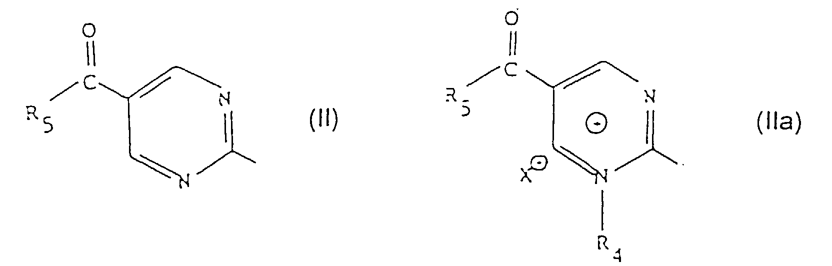 worin R₄ für einen (C₁-C₄)-Alkyl-, (C₇-C₉)-Aralkyl-, Phenyl-Rest oder ein Wasserstoffatom, und R₅ für einen Hydroxy-, (C₁-C₄)-Alkoxy-, oder Aminorest und X^{⊖} für ein pharmakologisch akzeptables Kation stehen oder
worin R₂, R₃ und R₄ Wasserstoffatome darstellen oder R₂, R₃ und R₄ gemeinsam zusammen mit den Stickstoffatomen, an die sie gebunden sind, und dem Kohlenstoffatom, an welches die genannten Stickstoffatome gebunden sind, einen Imidazol-Ring der Formel 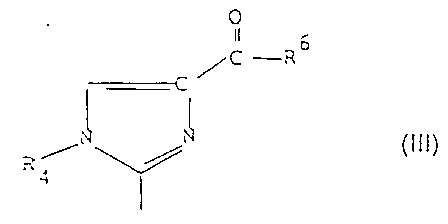 oder einen 1,6-Dihydropyrimidin-Ring der Formel: 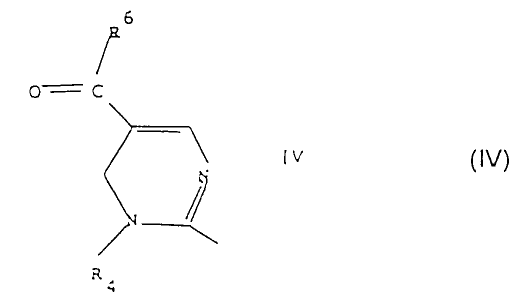 bilden, worin R₆ eine der für R₅ angegebenen Bedeutungen hat, oder
worin R₂ und R₃ gemeinsam zusammen mit den Stickstoffatomen, an die sie gebunden sind, und dem Kohlenstoffatom, an das die genannten Stickstoffatome gebunden sind, einen Ring der Formel bilden: 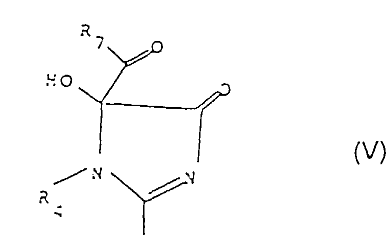 worin R₇ einen (C₁-C₄)-Alkoxy- oder 1-Glyceryl-Rest darstellt, wobei im zuletzt genannten Fall R₁ einem Wasserstoffatom entsprechen kann, und die pharmazeutisch akzeptablen Salze dieser Verbindungen mit Ausnahme von Veratrylguanidin und des Hemisulfats desselben und der Verbindungen der Formel (I), worin R₂, R₃ und R₄ Wasserstoffatome darstellen und R₁ ein Benzylrest ist, der in m- und p-Stellung gleichzeitig durch zwei Ethoxy- oder zwei n-Propoxy-Reste substituiert ist, und der Hemisulfate derselben und mit Ausnahme der Verbindung der Formel (I), worin R₂, R₃ und R₄ für ein Wasserstoffatom und R₁ für -(CH₂)₃-CH(COOH)-NH-COOtBu stehen.

2. Verbindungen nach Anspruch 1, dadurch gekennzeichnet, daß R₁ einen Isopropylrest oder einen Di- oder Trimethoxybenzylrest oder den Rest bedeutet

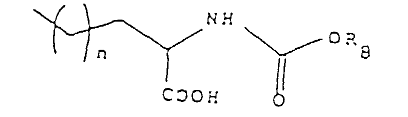

3. Verbindungen nach Anspruch 1, dadurch gekennzeichnet, daß R₄ ein Methyl-, Ethyl-, n-Propyl- oder Benzylrest ist.

4. Verbindungen nach einem der Ansprüche 1 bis 3, dadurch gekennzeichnet, daß sie der Formel entsprechen 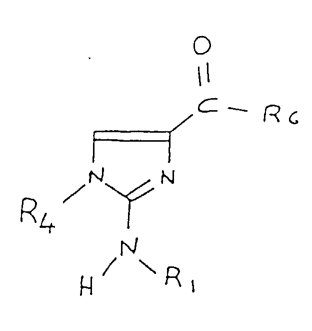 sowie die pharmazeutisch akzeptablen Salze dieser Verbindungen.

5. Verbindung nach Anspruch 4, dadurch gekennzeichnet, daß es sich dabei um 1-Methyl-2-isopropylamino-4-carboxy-imidazol handelt.

6. Verbindungen nach Anspruch 1, dadurch gekennzeichnet, daß sie ausgewählt werden aus dem Lactat, Fumarat, Hydrochlorid, Maleat, Malat, Ketoglutarat, Glutarat, Phenoxyacetat, Sulfonat, Picrat, Tartrat und Methansulfonat von Veratrylguanidin.

7. Verbindungen der Formel (I) nach einem der Ansprüche 1 bis 6 und Verbindung der Formel (I), worin R₂, R₃ und R₄ Wasserstoffatome und R₁ -(CH₂)₃-CH(COOH)-NH-COOtBu darstellen, zur Verwendung als therapeutisch aktive Substanz.

8. Verbindungen der Formel (I) nach Anspruch 7, dadurch gekennzeichnet, daß sie für die Behandlung von Neuropathien oder Myopathien angewendet werden.

9. Pharmazeutische Zusammensetzung, die mindestens eine Verbindung nach Anspruch 7 oder 8 und einen pharmakologisch akzeptablen inerten Träger enthält.

10. Verwendung von Veratrylguanidin oder des Hemisulfats desselben oder der Verbindungen der Formel (I), worin R₂, R₃ und R₄ Wasserstoffatome und R₁ einen Benzylrest, der in m- und p-Stellung gleichzeitig durch zwei Ethoxy- oder 2 n-Propoxyreste substituiert ist, darstellen, und der Hemisulfate derselben zur Herstellung eines Arzneimittels für die Behandlung von Neuropathien oder Myopathien.

11. Verfahren zur Herstellung der Verbindungen der Formel (I) nach einem der Ansprüche 1 bis 3, in der R₂, R₃ und R₄ gemeinsam zusammen mit den Stickstoffatomen, an die sie gebunden sind, und dem Kohlenstoffatom, an das die genannten Stickstoffatome gebunden sind, einen Pyrimidinring der Formel (II) bilden,
das darin besteht, daß man ein Guanidinsäuresalz der Formel 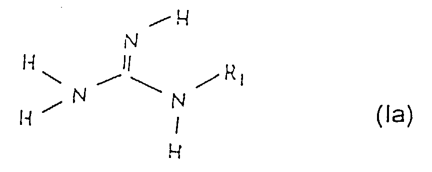 mit einem (C₁-C₄)-Alkoxycarbonylmalonaldehyd der Formel 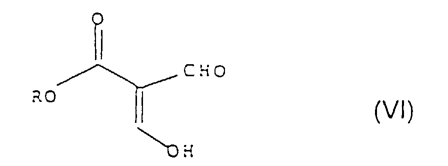 worin R für einen (C₁-C₄)-Alkylrest steht,
in Gegenwart eines Säureakzeptors und gegebenenfalls in Gegenwart eines protischen polaren Lösungsmittels in Kontakt bringt, wobei die erhaltenen Verbindungen gegebenenfalls hydrolysiert und gegebenenfalls in ein Carboxamid und/oder gegebenenfalls in ein 1-Alkylpyrimidinium-hydroxid der Formel (IIa) umgewandelt werden können durch Einwirkenlassen eines Dialkylsulfats, und die anschließend in ein anderes Salz überführt werden können.

12. Verfahren zur Herstellung der Verbindungen der Formel (I) nach einem der Ansprüche 1 bis 3, in der R₂, R₃ und R₄ gemeinsam zusammen mit den Stickstoffatomen, an die sie gebunden sind, und dem Kohlenstoffatom, an das die genannten Stickstoffatome gebunden sind, einen 1,6-Dihydropyrimidin-Ring der Formel (IV) bilden,
das für den Fall, daß R₄ einen Alkylrest darstelt, darin besteht, daß man das Pyrimidiniumhydroxid der Formel: 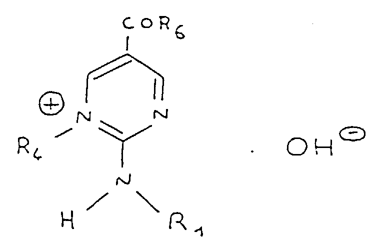 mit einem Reduktionsmittel wie Natriumtetraborhydrid in Kontakt bringt.

13. Verfahren zur Herstellung der Verbindungen der Formel (I) nach Anspruch 1, in der R₂, R₃ und R₄ gemeinsam zusammen mit den Stickstoffatomen, an die sie gebunden sind, und dem Kohlenstoffatom, an das die genannten Stickstoffatome gebunden sind, einen Imidazolring der Formel (III) bilden, dadurch gekennzeichnet, daß es im wesentlichen darin besteht, die den Verbindungen der Formel (III) entsprechenden 4,5-Dicarboxyimidazole durch Erwärmen, gegebenenfalls in Gegenwart eines polaren Lösungsmittels, partiell zu decarboxylieren.

14. Verfahren zur Herstellung der Verbindungen der Formel (I) nach Anspruch 1, in der R₂, R₃ und R₄ gemeinsam zusammen mit den Stickstoffatomen, an die sie gebunden sind, und dem Kohlenstoffatom, an das die genannten Stickstoffatome gebunden sind, einen Imidazolring der Formel (III) bilden, worin R₄ ein Wasserstoffatom darstellt,
dadurch gekennzeichnet, daß es im wesentlichen darin besteht, daß man eine Verbindung der folgenden Formel dehydratisiert:

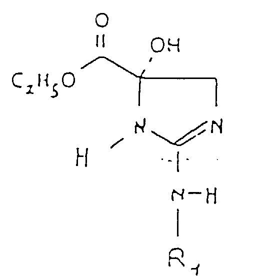

## Claims

1. Compounds of formula: 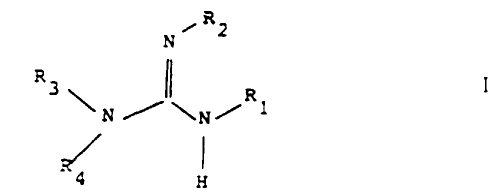 in which:
R₁ is an isopropyl radical or a benzyl radical which is optionally substituted by one or more (C₁-C₄)alkoxy radicals or a radical: 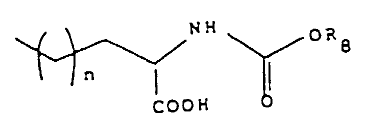
R₂, R₃ and R₄ together with the nitrogen atoms to which they are attached and the carbon atom to which the said nitrogen atoms are attached form a pyrimidine ring of formula II or a pyrimidinium ring of formula IIa: 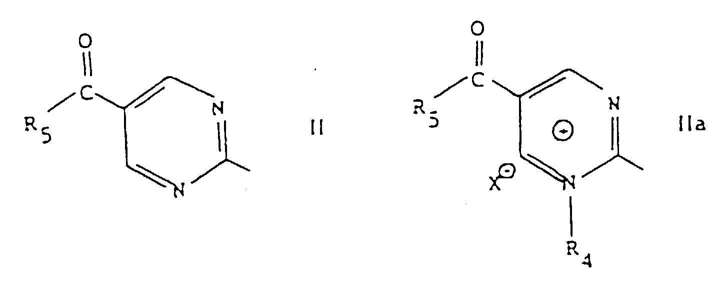 in which
n = 0, 1 or 2,
R₄ is a (C₁-C₄)alkyl radical, a (C₇-C₉)aralkyl radical, a phenyl radical or a hydrogen atom,
R₈ is a (C₁-C₄)alkyl radical or a (C₇-C₉)aralkyl radical,
R₅ is a hydroxyl radical, a (C₁-C₄)alkoxy radical or an amino radical
X⁻ is a pharmacologically acceptable cation
or R₂, R₃ and R₄ are a hydrogen atom,
or R₂, R₃ and R₄ together form with the nitrogen atoms to which they are attached and the carbon atom to which the said nitrogen atoms are attached an imidazole ring of formula: 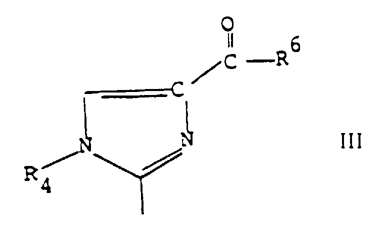 or 1,6-dihydropyrimidine of formula: 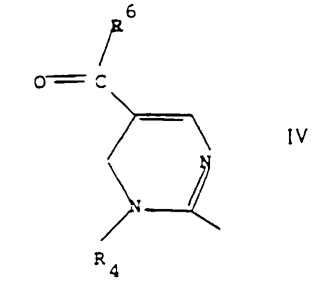
R₆ having one of the meanings of R₅,
or R₂, R₃ together form with the nitrogen atoms to which they are attached and the carbon atom to which the said nitrogen atoms are attached a ring of formula: 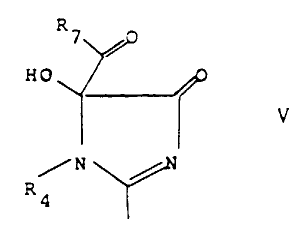
R₇ being a (C₁-C₄)alkoxy radical or a 1-glyceryl radical with in this latter case R₁ which may correspond to a hydrogen atom,
and the pharmacologically acceptable salts of these compounds,
with the exception of veratrylguanidine and the hemisulfate thereof and of the compounds of formula I wherein R₂, R₃, R₄ are hydrogen atoms and R₁ is a benzyl radical substituted in the meta and para positions simultaneously by two ethoxy radicals or two n-propoxy radicals and of the hemisulphates thereof,
and with the exception of the compound wherein R₂, R₃, R₄ are hydrogen atoms and R₁ is -(CH₂)₃-CH(COOH)-NH-COOtBu.

2. Compounds according to Claim 1, characterized in that R₁ is an isopropyl radical or a di- or trimethoxybenzyl radical or the radical:

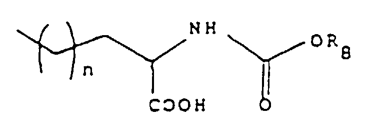

3. Compounds according to Claim 1, characterized in that R₄ is a methyl, ethyl, n-propyl or benzyl radical.

4. Compounds according to one of Claims 1 to 3, characterized in that they corespond to the formula 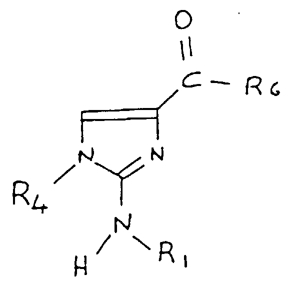 as well as the pharmaceutically acceptable salts of these compounds.

5. Compound according to Claim 4, characterized in that it is 1-methyl-2-isopropylamino-4-carboxyimidazole.

6. Compounds according to Claim 1, characterized in that they are chosen from veratrylguanidine lactate, fumarate, hydrochloride, maleate, malate, ketoglutarate, glutarate, phenoxyacetate, sulphonate, picrate, tartrate and methanesulphonate.

7. Compounds of formula I according to one of Claims 1 to 6, and the compound of formula I wherein R₂, R₃, R₄ are hydrogen atoms and R₁ is -(CH₂)₃-CH(COOH)-NH-COOtBu, for their application as therapeutically active substance.

8. Compounds of formula I according to Claim 7, characterized in that they are used for the treatment of neuropathies or of myopathies.

9. Pharmaceutical composition containing at least one compound according to Claim 7 or 8 and a pharmacologically acceptable inert carrier.

10. Use of veratrylguanidine or of the hemisulphate thereof or of the compounds of formula I wherein R₂, R₃, R₄ are hydrogen atoms and R₁ is a benzyl radical substituted in the meta and para positions simultaneously by two ethoxy radical or two n-propoxy radicals and the hemisulphates thereof for the manufacture of a medicinal product useful in the treatment of neuropathies or of myopathies.

11. Process for preparing the compounds of formula I according to one of Claims 1 to 3, in which R₂, R₃, R₄ together with the nitrogen atoms to which they are attached and the carbon atom to which the said nitrogen atoms are attached form a pyrimidine ring of formula II, consisting in placing an acid salt of guanidine of formula: 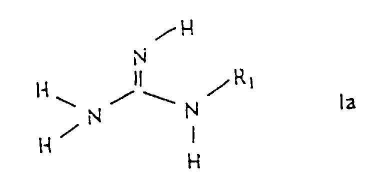 in contact with a (C₁-C₄) alkoxycarbonylmalonaldehyde of formula: 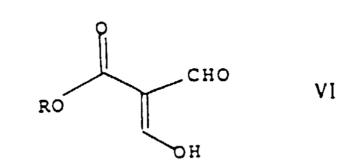 (wherein R is a (C₁-C₄)alkyl)
in the presence of an acid acceptor and optionally a protic polar solvent, it being possible for the compounds obtained to be optionally hydrolysed and optionally converted to carboxamide and/or optionally to 1-alkyl-pyrimidinium hydroxide of formula IIa by action of a dialkyl sulphate, and then converted to another salt.

12. Process for preparing the compounds of formula I according to one of Claims 1 to 3, in which R₂, R₃ and R₄ together form with the nitrogen atoms to which they are attached and the carbon atom to which the said nitrogen atoms are attached a 1,6-dihydropyrimidine ring of formula IV consisting in the case where R₄ is an alkyl radical in placing the pyrimidinium hydroxide of formula: 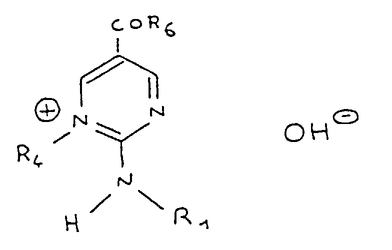 in contact with a reducing agent such as sodium tetraborohydride.

13. Process for preparing the compounds of formula I according to Claim 1, in which R₂, R₃ and R₄ together form with the nitrogen atoms to which they are attached and the carbon atom to which the said nitrogen atoms are attached an imidazole ring of formula III, characterized in that it essentially consists in partially decarboxylating the 4,5-dicarboxyimidazoles corresponding to the compounds of formula III by heating, optionally in the presence of a polar solvent.

14. Process for preparing the compounds of formula I according to Claim 1, in which R₂, R₃ and R₄ together form with the nitrogen atoms to which they are attached and the carbon atom to which the said nitrogen atoms are attached, an imidazole ring of formula III wherein R₄ is a hydrogen atom, characterized in that it essentially consists in dehydrating a compound of formula:
